# EUROPEAN PATENT SPECIFICATION

(11) **EP 3 902 594 B1**
(45) Date of publication and mention of the grant of the patent: **20.11.2024**
(21) Application number: 18836582.9
(22) Date of filing: 28.12.2018
(51) Int. Cl.: A61N 1/05, A61N 1/36, A61B 5/12

(54) **SYSTEMS FOR MONITORING OF EVOKED RESPONSES THAT OCCUR DURING AN ELECTRODE LEAD INSERTION PROCEDURE**
SYSTEME ZUR ÜBERWACHUNG VON EVOZIERTEN REAKTIONEN, DIE WÄHREND EINES ELEKTRODENKABELEINSETZVERFAHRENS AUFTRETEN
SYSTÈMES DE SURVEILLANCE DE RÉPONSES ÉVOQUÉES APPARAISSANT LORS D'UNE PROCÉDURE D'INSERTION D'UN FIL D'ÉLECTRODE

(43) Date of publication of application: 03.11.2021
(73) Proprietor: Advanced Bionics AG, 8712 Staefa (CH)
(72) Inventor: KOESTER, Kurt, J., Los Angeles, CA 90041 (US); KOKA, Kanthaiah, Valencia, CA 91355 (US); LITVAK, Leonid, M., Los Angeles, CA 90038 (US); EMCH, Hansjuerg, 8006 Zuerich (CH)
(74) Representative: Schwan Schorer & Partner mbB
(86) International application number: PCT/US2018/068054
(87) International publication number: WO 2020/139397

(56) References cited:
- WO-A1-2013/142846
- WO-A1-2017/065809
- WO-A1-2017/131675
- WO-A1-2018/037312
- US-A1- 2015 118 658
- US-A1- 2015 314 122
- US-A1- 2016 059 014

## Description

### BACKGROUND INFORMATION

During an insertion procedure in which an electrode lead is placed within the cochlea, it may be desirable to monitor evoked responses (e.g., electrocochleographic ("ECoG" or "ECochG") potentials) that occur within the recipient in response to acoustic stimulation applied to the recipient. These evoked responses may be indicative of electrode positioning within the cochlea, trauma that may occur to the cochlea during the insertion procedure, residual hearing of different areas of the cochlea as the electrode lead is inserted, and/or various other factors associated with the insertion procedure.
WO 2017/131675 A1 relates to a system for monitoring insertion of a cochlear lead into a cochlea based on evoked response measurements at an electrode of the cochlear lead, wherein an audio sound alert is provided when a change between a first evoked response and a second evoked response is found to be greater than a predetermined threshold. WO 2013/142846 A1 relates to a similar system, wherein a beep alert is provided when the evoked response is found to be not within a predetermined range of a baseline response and/or one or more previously recorded evoked responses. Another system for monitoring insertion of a cochlear lead into a cochlea is known rom WO 2018/037312 A1. US 2015/01 18658A1 relates to an adaptive interface for a continuous patient monitoring device, providing for audio feedback wherein frequency, tone and volume of an alarm may be changed.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 illustrates an exemplary cochlear implant system according to principles described herein.
FIG. 2 illustrates a schematic structure of the human cochlea according to principles described herein.
FIG. 3 illustrates an exemplary diagnostic system according to principles described herein.
FIG. 4 illustrates an exemplary stand-alone diagnostic system according to principles described herein.
FIG. 5 shows a base module detached from a computing module according to principles described herein.
FIGS. 6-8 depict exemplary configurations in which a diagnostic system is used to perform one or more diagnostic operations during a surgical procedure involving an electrode lead according to principles described herein.
FIGS. 9A-12 illustrate an exemplary hardware implementation of the diagnostic system of FIG. 4 according to principles described herein.
FIGS. 13A-13B illustrate different mappings that may be performed by diagnostic system according to principles described herein.
FIG. 14 illustrates an exemplary graphical user interface according to principles described herein.
FIG. 15 illustrates an exemplary graphical user interface according to principles described herein.
FIG. 16 illustrates an exemplary method according to principles described herein.
FIG. 17 illustrates an exemplary computing device according to principles described herein.

### DETAILED DESCRIPTION

The invention relates to a system for monitoring of evoked responses that occur during an electrode lead insertion procedure as defined in claim 1. For example, a diagnostic system may determine a minimum evoked response amplitude and a maximum evoked response amplitude for a recipient of a cochlear implant. The diagnostic system may determine a mapping between a plurality of audible pitches and a plurality of evoked response amplitudes included in a range defined by the minimum and maximum evoked response amplitudes. The diagnostic system may monitor, during an insertion procedure in which an electrode lead communicatively coupled to the cochlear implant is inserted into a cochlea of the recipient, an evoked response signal recorded during the insertion procedure by an electrode disposed on the electrode lead. The evoked response signal represents amplitudes of a plurality of evoked responses that occur within the recipient in response to acoustic stimulation applied to the recipient. The evoked responses may each be an ECoG potential (e.g., a cochlear microphonic potential, an action potential, a summating potential, etc.), an auditory nerve response, a brainstem response, a compound action potential, a stapedius reflex, and/or any other type of neural or physiological response that may occur within a recipient in response to application of acoustic stimulation to the recipient. Evoked responses may originate from neural tissues, hair cell to neural synapses, inner or outer hair cells, or other sources.

As the evoked response signal is being monitored, the diagnostic system may detect an amplitude change in the evoked response signal and present, based on the mapping, acoustic feedback that audibly indicates the amplitude change.

To illustrate, the mapping may specify that a first audible pitch is mapped to a first evoked response amplitude and that a second audible pitch is mapped to a second evoked response amplitude that is different than the first evoked response amplitude. During the insertion procedure, the diagnostic system may direct an acoustic stimulation generator to apply acoustic stimulation to the recipient. The diagnostic system may detect a first evoked response that occurs at a first time within the recipient in response to the acoustic stimulation (e.g., by directing the cochlear implant to use the electrode disposed on the electrode lead to detect the first evoked response). In response to detecting the first evoked response, the diagnostic system may present, based on the mapping, acoustic feedback (e.g., an audible tone) that has the first audible pitch to a user of the diagnostic system. The diagnostic system may direct the acoustic stimulation generator to continue applying the acoustic stimulation to the recipient. As the electrode lead is inserted further into the cochlea, the diagnostic system may detect a second evoked response that occurs at a second time within the recipient in response to the acoustic stimulation. In response to detecting the second evoked response, the diagnostic system may present, based on the mapping, acoustic feedback that has the second audible pitch to the user of the diagnostic system.

In some examples, the systems and methods described herein are implemented by a stand-alone diagnostic system that includes a computing module and a base module configured to attach to the computing module (e.g., a back side of the computing module) and serve as a stand for the computing module. The computing module includes a display screen and a processor configured to direct the display screen to display a graphical user interface. The base module houses an interface unit configured to be communicatively coupled to the processor and to a cochlear implant while the base module is attached to the computing module. In this configuration, the processor may be configured to detect a request to begin monitoring an evoked response signal associated with a recipient of the cochlear implant during an insertion procedure in which an electrode lead is inserted into a cochlea of the recipient. In response to the request, the processor may direct the interface unit to apply acoustic stimulation to the recipient by way of a sound delivery apparatus coupled to the base module and direct the cochlear implant to record the evoked response signal using an electrode disposed on the electrode lead. In response to detecting an amplitude change in the evoked response signal, the processor may present acoustic feedback that audibly indicates the amplitude change.

By providing acoustic feedback that audibly indicates amplitude changes in the evoked response signal recorded by the electrode during the electrode lead insertion procedure, the systems and methods described herein may advantageously allow a surgeon or other user involved with the insertion procedure to readily ascertain various characteristics and/or events associated with the insertion procedure without having to look at a display screen that shows information associated with the insertion procedure. This may allow the surgeon to focus his or her visual attention on the insertion procedure itself (e.g., by focusing his or her eyes on a surgical scope) while still receiving feedback representative of the characteristics and/or events associated with the insertion procedure.

For example, during an electrode lead insertion procedure, relatively low frequency (e.g., 500 Hz) acoustic stimulation may be applied to the recipient. This relatively low frequency corresponds to a location that is relatively deep within the cochlea (i.e., close to the apex of the cochlea). As such, as the surgeon advances the electrode lead further into the cochlea, the evoked response amplitude recorded by the electrode on the electrode lead will increase as long as the electrode lead is being properly advanced within the cochlea. The audible pitch of the acoustic feedback may correspondingly increase, thereby indicating to the surgeon that the electrode lead is being properly advanced within the cochlea. However, at some point, the electrode lead may damage a wall or other structure of the cochlea. This may cause the evoked responses detected by the electrode on the electrode lead to suddenly decrease in amplitude. A corresponding decrease in audible pitch of the acoustic feedback being presented by the diagnostic system to the surgeon (and, in some, examples, an additional type of acoustic feedback is presented that does not include any of the audible pitches used in the mapping) may immediately make the surgeon aware of this sudden decrease in evoked response amplitude. The surgeon may then take remedial action (e.g., by stopping the lead insertion, repositioning or redirecting the electrode lead within the cochlea, etc.).

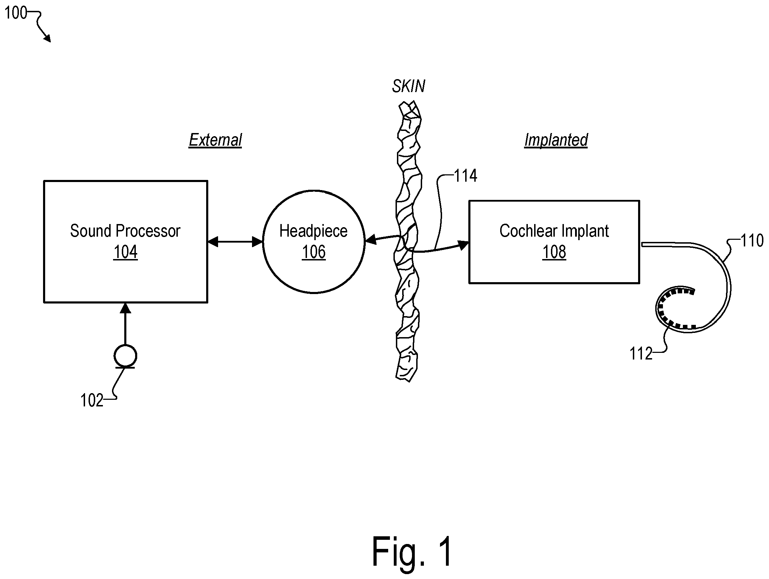

FIG. 1 illustrates an exemplary cochlear implant system 100. As shown, cochlear implant system 100 may include a microphone 102, a sound processor 104, a headpiece 106 having a coil disposed therein, a cochlear implant 108, and an electrode lead 110. Electrode lead 110 may include an array of electrodes 112 disposed on a distal portion of electrode lead 110 and that are configured to be inserted into a cochlea of a recipient to stimulate the cochlea when the distal portion of electrode lead 110 is inserted into the cochlea. One or more other electrodes (e.g., including a ground electrode, not explicitly shown) may also be disposed on other parts of electrode lead 110 (e.g., on a proximal portion of electrode lead 110) to, for example, provide a current return path for stimulation current generated by electrodes 112 and to remain external to the cochlea after electrode lead 110 is inserted into the cochlea. As shown, electrode lead 110 may be pre-curved so as to properly fit within the spiral shape of the cochlea. Additional or alternative components may be included within cochlear implant system 100 as may serve a particular implementation.

As shown, cochlear implant system 100 may include various components configured to be located external to a recipient including, but not limited to, microphone 102, sound processor 104, and headpiece 106. Cochlear implant system 100 may further include various components configured to be implanted within the recipient including, but not limited to, cochlear implant 108 and electrode lead 110.

Microphone 102 may be configured to detect audio signals presented to the user. Microphone 102 may be implemented in any suitable manner. For example, microphone 102 may include a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 104. Additionally or alternatively, microphone 102 may be implemented by one or more microphones disposed within headpiece 106, one or more microphones disposed within sound processor 104, one or more beam-forming microphones, and/or any other suitable microphone as may serve a particular implementation.

Sound processor 104 may be configured to direct cochlear implant 108 to generate and apply electrical stimulation (also referred to herein as "stimulation current") representative of one or more audio signals (e.g., one or more audio signals detected by microphone 102, input by way of an auxiliary audio input port, input by way of a clinician's programming interface (CPI) device, etc.) to one or more stimulation sites associated with an auditory pathway (e.g., the auditory nerve) of the recipient. Exemplary stimulation sites include, but are not limited to, one or more locations within the cochlea, the cochlear nucleus, the inferior colliculus, and/or any other nuclei in the auditory pathway. To this end, sound processor 104 may process the one or more audio signals in accordance with a selected sound processing strategy or program to generate appropriate stimulation parameters for controlling cochlear implant 108. Sound processor 104 may be housed within any suitable housing (e.g., a behind-the-ear ("BTE") unit, a body worn device, headpiece 106, and/or any other sound processing unit as may serve a particular implementation).

In some examples, sound processor 104 may wirelessly transmit stimulation parameters (e.g., in the form of data words included in a forward telemetry sequence) and/or power signals to cochlear implant 108 by way of a wireless communication link 114 between headpiece 106 and cochlear implant 108 (e.g., a wireless link between a coil disposed within headpiece 106 and a coil physically coupled to cochlear implant 108). It will be understood that communication link 114 may include a bi-directional communication link and/or one or more dedicated uni-directional communication links.

Headpiece 106 may be communicatively coupled to sound processor 104 and may include an external antenna (e.g., a coil and/or one or more wireless communication components) configured to facilitate selective wireless coupling of sound processor 104 to cochlear implant 108. Headpiece 106 may additionally or alternatively be used to selectively and wirelessly couple any other external device to cochlear implant 108. To this end, headpiece 106 may be configured to be affixed to the recipient's head and positioned such that the external antenna housed within headpiece 106 is communicatively coupled to a corresponding implantable antenna (which may also be implemented by a coil and/or one or more wireless communication components) included within or otherwise associated with cochlear implant 108. In this manner, stimulation parameters and/or power signals may be wirelessly transmitted between sound processor 104 and cochlear implant 108 via communication link 114.

Cochlear implant 108 may include any suitable type of implantable stimulator. For example, cochlear implant 108 may be implemented by an implantable cochlear stimulator. Additionally or alternatively, cochlear implant 108 may include a brainstem implant and/or any other type of cochlear implant that may be implanted within a recipient and configured to apply stimulation to one or more stimulation sites located along an auditory pathway of a recipient.

In some examples, cochlear implant 108 may be configured to generate electrical stimulation representative of an audio signal processed by sound processor 104 (e.g., an audio signal detected by microphone 102) in accordance with one or more stimulation parameters transmitted thereto by sound processor 104. Cochlear implant 108 may be further configured to apply the electrical stimulation to one or more stimulation sites (e.g., one or more intracochlear regions) within the recipient via electrodes 112 disposed along electrode lead 110. In some examples, cochlear implant 108 may include a plurality of independent current sources each associated with a channel defined by one or more of electrodes 112. In this manner, different stimulation current levels may be applied to multiple stimulation sites simultaneously by way of multiple electrodes 112.

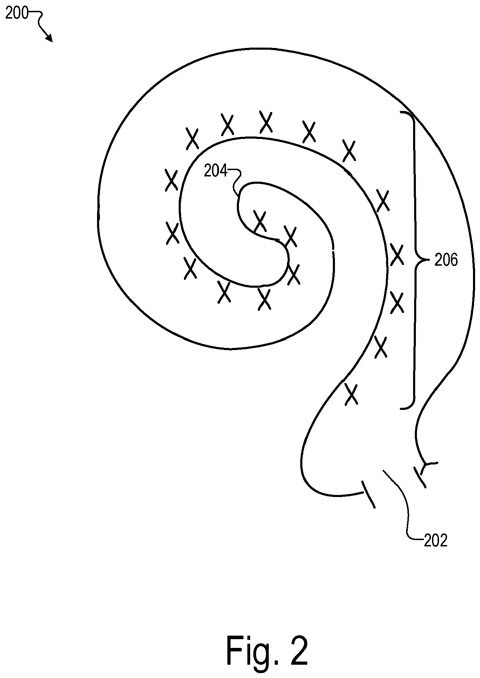

FIG. 2 illustrates a schematic structure of the human cochlea 200 into which electrode lead 110 may be inserted. As shown in FIG. 2, cochlea 200 is in the shape of a spiral beginning at a base 202 and ending at an apex 204. Within cochlea 200 resides auditory nerve tissue 206, which is denoted by Xs in FIG. 2. The auditory nerve tissue 206 is organized within the cochlea 200 in a tonotopic manner. Relatively low frequencies are encoded at or near the apex 204 of the cochlea 200 (referred to as an "apical region") while relatively high frequencies are encoded at or near the base 202 (referred to as a "basal region"). Hence, electrical stimulation applied by way of electrodes disposed within the apical region (i.e., "apical electrodes") may result in the recipient perceiving relatively low frequencies and electrical stimulation applied by way of electrodes disposed within the basal region (i.e., "basal electrodes") may result in the recipient perceiving relatively high frequencies. The delineation between the apical and basal electrodes on a particular electrode lead may vary depending on the insertion depth of the electrode lead, the anatomy of the recipient's cochlea, and/or any other factor as may serve a particular implementation.

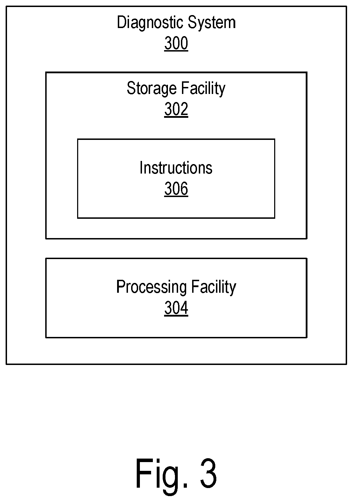

FIG. 3 illustrates an exemplary diagnostic system 300 that may be configured to perform any of the operations described herein. As shown, diagnostic system 300 may include, without limitation, a storage facility 302 and a processing facility 304 selectively and communicatively coupled to one another. Facilities 302 and 304 may each include or be implemented by hardware and/or software components (e.g., processors, memories, communication interfaces, instructions stored in memory for execution by the processors, etc.). In some examples, facilities 302 and 304 may be distributed between multiple devices and/or multiple locations as may serve a particular implementation.

Storage facility 302 may maintain (e.g., store) executable data used by processing facility 304 to perform any of the operations described herein. For example, storage facility 302 may store instructions 306 that may be executed by processing facility 304 to perform any of the operations described herein. Instructions 306 may be implemented by any suitable application, software, code, and/or other executable data instance. Storage facility 302 may also maintain any data received, generated, managed, used, and/or transmitted by processing facility 304.

Processing facility 304 may be configured to perform (e.g., execute instructions 306 stored in storage facility 302 to perform) various operations associated with monitoring evoked responses that occur within a recipient of a cochlear implant during an electrode lead insertion procedure in which an electrode lead is inserted into a cochlea of the recipient. For example, processing facility 304 may determine a minimum evoked response amplitude and a maximum evoked response amplitude for a recipient of a cochlear implant and determine a mapping between a plurality of audible pitches and a plurality of evoked response amplitudes included in a range defined by the minimum and maximum evoked response amplitudes. Processing facility 304 may also monitor, during an insertion procedure in which an electrode lead communicatively coupled to the cochlear implant is inserted into a cochlea of the recipient, an evoked response signal recorded during the insertion procedure by an electrode disposed on the electrode lead, the evoked response signal representing amplitudes of a plurality of evoked responses that occur within the recipient in response to acoustic stimulation applied to the recipient. Processing facility 304 may detect an amplitude change in the evoked response signal as the evoked response signal is being monitored and present, based on the mapping and as the evoked response signal is being monitored, acoustic feedback that audibly indicates the amplitude change. These operations are described in more detail herein.

Diagnostic system 300 may be implemented in any suitable manner. For example, diagnostic system 300 may be implemented by a stand-alone diagnostic system that may be used in a surgical operating room to perform any of the operations described herein.

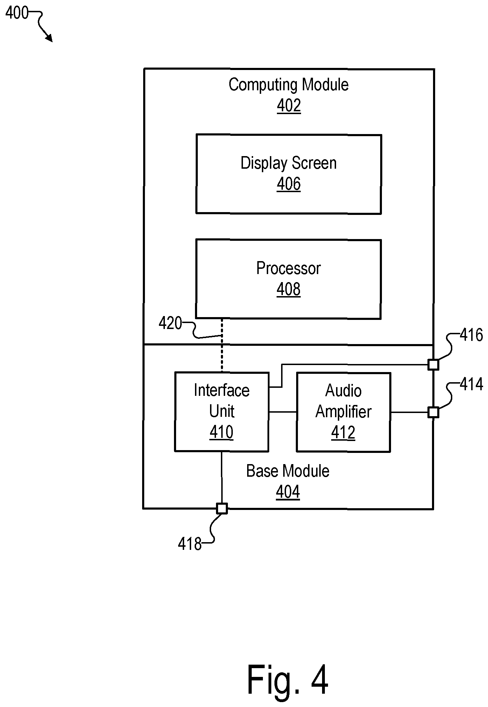

FIG. 4 illustrates an exemplary stand-alone diagnostic system 400 that may implement diagnostic system 300. As shown, diagnostic system 400 includes a computing module 402 and a base module 404. Computing module 402 includes a display screen 406 and a processor 408. Base module 404 includes an interface unit 410, an audio amplifier 412, an audio output port 414, a communications port 416, and a port 418. Computing module 402 and base module 404 may include additional or alternative components as may serve a particular implementation. For example, computing module 402 and/or base module 404 may include one or more speakers configured to output acoustic feedback and/or other types of sound configured to be heard by a surgeon and/or other user of diagnostic system 400. Diagnostic system 400 and exemplary implementations thereof are described more fully in co-pending PCT Application No. PCT/US18/67900, which application is filed the same day as the present application and incorporated herein by reference in its entirety.

In the configuration shown in FIG. 4, base module 404 is physically attached to computing module 402. In this configuration, processor 408 is communicatively coupled to interface unit 410 by way of a connection 420. Connection 420 may be implemented by any suitable connection (e.g., an internal USB connection) as may serve a particular implementation. As will be described in more detail below, base module 404 may be selectively detached from computing module 402 and connected to a different computing device by way of port 418.

Display screen 406 may be configured to display any suitable content associated with an application executed by processor 408. Display screen 406 may be implemented by a touchscreen and/or any other type of display screen as may serve a particular implementation.

Processor 408 may be configured to execute a diagnostic application associated with a cochlear implant (e.g., cochlear implant 108). For example, processor 408 may execute a diagnostic application that may be used during a surgical procedure associated with the cochlear implant. The diagnostic application may be configured to perform various diagnostic operations associated with the cochlear implant during the surgical procedure. Exemplary diagnostic operations are described herein.

In some examples, processor 408 may direct display screen 406 to display a graphical user interface associated with the diagnostic application being executed by processor 408. A user may interact with the graphical user interface to adjust one or more parameters associated with the cochlear implant and/or otherwise obtain information that may be useful during a procedure associated with the cochlear implant.

Base module 404 may be configured to attach to computing module 402 and serve as a stand for computing module 402.

Interface unit 410 is configured to be communicatively coupled to processor 408 by way of connection 420 while base module 404 is attached to computing module 402. Interface unit 410 is further configured to be communicatively coupled to the cochlear implant while base module 404 is attached to computing module 402. In this manner, interface unit 410 provides an interface between processor 408 and the cochlear implant.

Interface unit 410 may be communicatively coupled to the cochlear implant by way of communications port 416. For example, communications port 416 may be selectively coupled to a coil (e.g., a coil included in a headpiece, such as headpiece 106, or a disposable stand-alone coil) configured to wirelessly communicate with the cochlear implant. Interface unit 410 may communicate with the cochlear implant by transmitting and/or receiving data to/from the cochlear implant by way of the coil connected to communications port 416.

Interface unit 410 may be further configured to generate and provide acoustic stimulation (e.g., sound waves) to the recipient of the cochlear implant. To this end, audio output port 414 is configured to be selectively coupled to a sound delivery apparatus. In some examples, the sound delivery apparatus may be implemented by tubing that has a distal portion configured to be placed in or near an entrance to an ear canal of a recipient of the cochlear implant. While the sound delivery apparatus is connected to audio output port 414, interface unit 410 may transmit the acoustic stimulation to the recipient by way of the sound delivery apparatus.

As shown, audio amplifier 412 may be positioned within a path between interface unit 410 and audio output port 414. In this configuration, audio amplifier 412 may be configured to amplify the acoustic stimulation before the acoustic stimulation is delivered to the recipient by way of audio output port 414 and the sound delivery apparatus. In some alternative examples, amplification of the acoustic stimulation generated by interface unit 410 is not necessary, thereby obviating the need for audio amplifier 412 to be included in base module 404. Hence, in some implementations, base module 404 does not include audio amplifier 412.

In some examples, diagnostic system 400 may be configured to self-calibrate and/or perform in-situ testing. For example, processor 408 may calibrate an amplitude level of acoustic stimulation generated by interface unit 410 before and/or during a surgical procedure. Such self-calibration and in-situ testing may be performed in any suitable manner.

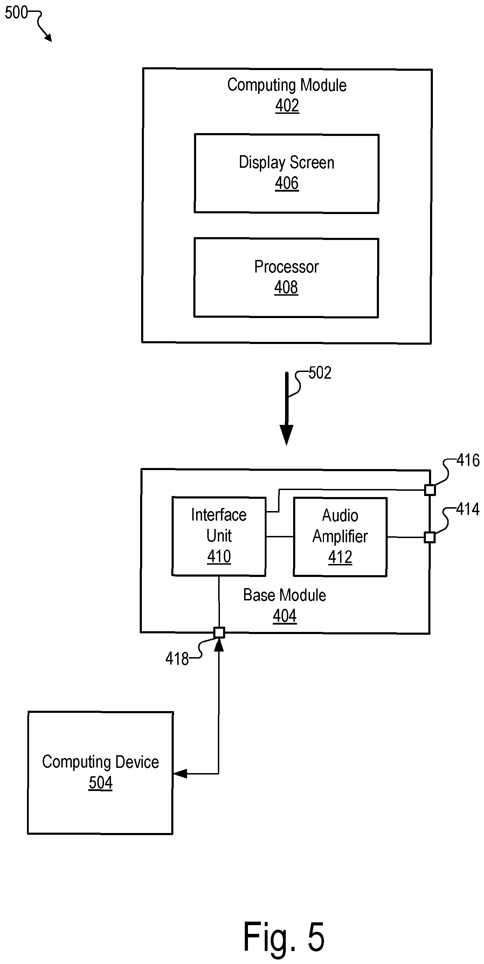

As mentioned, base module 404 may be selectively detached from computing module 402. To illustrate, FIG. 5 shows a configuration 500 in which base module 404 is detached from computing module 402. This detachment is illustrated by arrow 502. While detached, interface unit 410 of base module 404 may be communicatively coupled to a computing device 504. For example, interface unit 410 may be communicatively coupled to computing device 504 by plugging a cable (e.g., a USB cable) into port 418 and into computing device 504. In this configuration, computing device 504 may use interface unit 410 to interface with a cochlear implant (e.g., by providing acoustic stimulation to a recipient of the cochlear implant and/or receiving recording data from the cochlear implant).

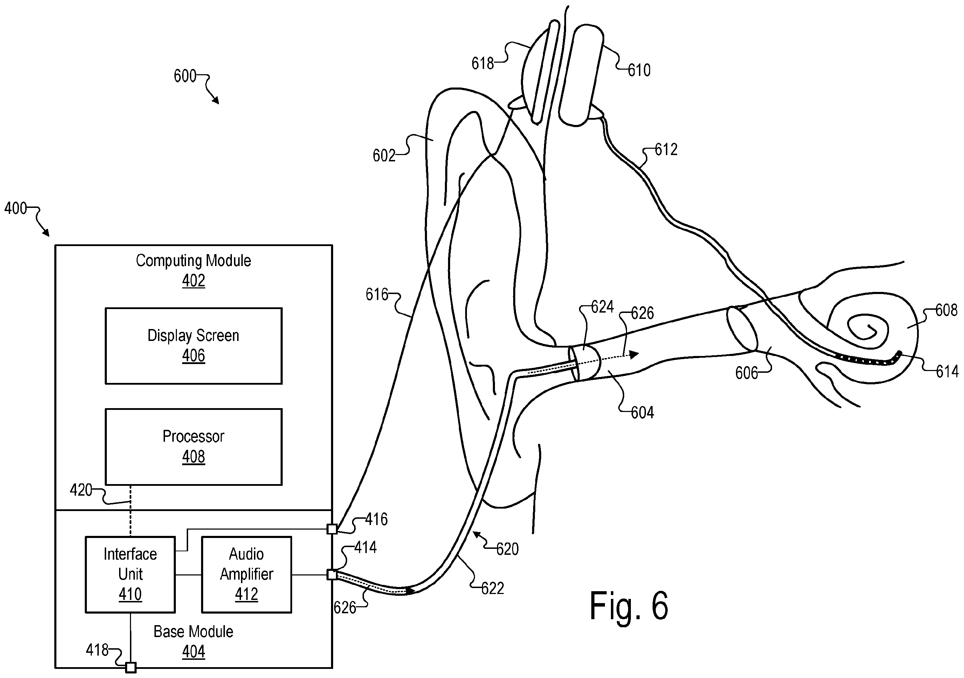

FIG. 6 depicts an exemplary configuration 600 in which diagnostic system 400 is used to perform one or more diagnostic operations during a surgical procedure involving a cochlear implant and an electrode lead. The surgical procedure may include, for example, an insertion procedure in which the cochlear implant is inserted into an incision pocket formed within the recipient and/or in which a distal portion of the electrode lead is positioned within the cochlea.

Various anatomical features of the recipient's ear are shown in FIG. 6. Specifically, anatomical features include a pinna 602 (i.e., the outer ear), an ear canal 604, a middle ear 606, and a cochlea 608. While no specific incision or other explicit surgical representation is shown in FIG. 6, it will be understood that such elements may be present when a surgical procedure is ongoing. For example, an incision may be present to allow the surgeon internal access to the recipient to insert the lead into cochlea 608. In some procedures, pinna 602 may be taped down and covered with surgical drapes so as to cover ear canal 604 (e.g., to help prevent fluids from reaching ear canal 604).

In the example of FIG. 6, a cochlear implant 610 and an electrode lead 612 are shown to be implanted within the recipient. Cochlear implant 610 may be similar, for example, to cochlear implant 108, and electrode lead 612 may be similar, for example, to electrode lead 110. Electrode lead 612 includes a plurality of electrodes (e.g., electrode 614, which is the distal-most electrode disposed on electrode lead 612).

As shown, a cable 616 of a headpiece 618 is connected to communications port 416. In this configuration, interface unit 410 may wirelessly communicate with cochlear implant 610 by way of a coil and/or other electronics included in headpiece 618, which may be similar to headpiece 106.

As also shown, a sound delivery apparatus 620 is connected to audio output port 414. Sound delivery apparatus 620 includes tubing 622 and an ear insert 624. Ear insert 624 is configured to fit at or within an entrance of ear canal 604. Tubing 622 and ear insert 624 together form a sound propagation channel 626 that delivers acoustic stimulation provided by interface unit 410 to the ear canal 604. Tubing 622 and ear insert 624 may be made out of any suitable material as may serve a particular implementation.

In some examples, processor 408 may execute a diagnostic application during the surgical procedure. In accordance with the diagnostic application, processor 408 may transmit, by way of connection 420, a command (also referred to as a stimulation command) to interface unit 410 for interface unit 410 to apply acoustic stimulation to the recipient and receive recording data representative of an evoked response that occurs within the recipient in response to the acoustic stimulation. In response to receiving the command, interface unit 410 may generate and apply the acoustic stimulation to the recipient by way of audio output port 414 and sound delivery apparatus 620. Interface unit 410 may also transmit a command (also referred to as a recording command) to cochlear implant 610 by way of communications port 416 and headpiece 618 for cochlear implant 610 to use electrode 614 to record the evoked response that occurs in response to the acoustic stimulation. Cochlear implant 610 may transmit the recording data back to interface unit 410 by way of headpiece 618 and communications port 416. Interface unit 410 may transmit the recording data to processor 408 by way of connection 420. Processor 408 may process the recording data and direct display screen 406 to display one or more graphical user interfaces associated with the recording data.

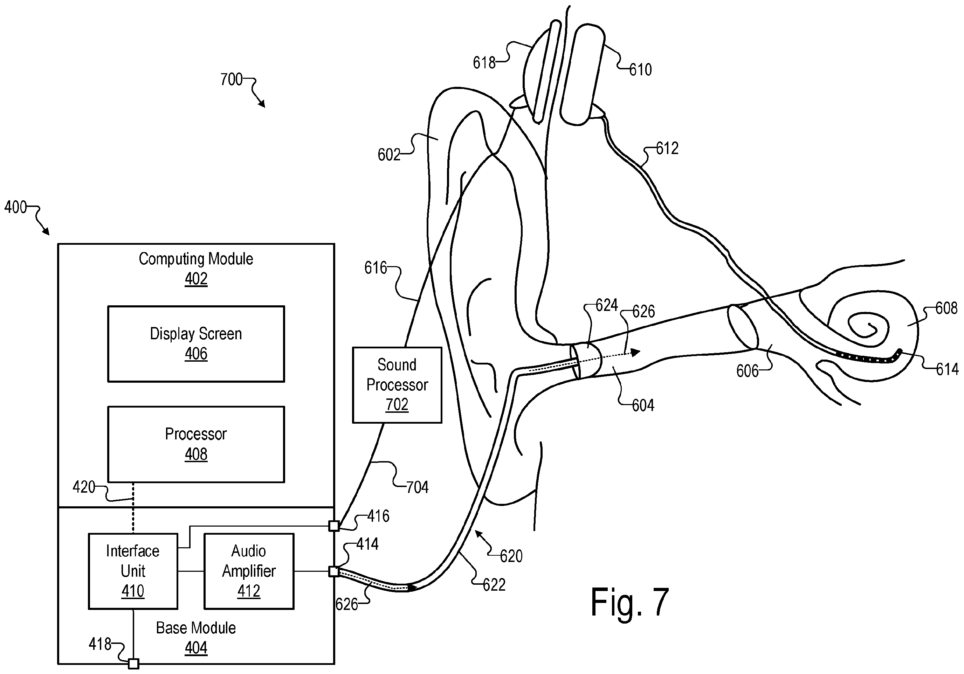

In configuration 600, headpiece 618 is connected directly to communications port 416 by way of cable 616. Hence, in configuration 600, interface unit 410 is configured to directly control cochlear implant 610. FIG. 7 illustrates an alternative configuration 700 in which a sound processor 702 is included in the communication path in between interface unit 410 and cochlear implant 610. Sound processor 702 may be similar to any of the sound processors (e.g., sound processor 104) described herein. In some examples, sound processor 702 is recipient-agnostic. In other words, sound processor 702 is not configured specifically for the recipient of cochlear implant 610. Rather, sound processor 702 may be used in a variety of different surgical procedures associated with a number of different recipients.

As shown, sound processor 702 is connected to communications port 416 by way of a cable 704. Sound processor 702 is also connected to headpiece 618 by way of cable 616. In this configuration, sound processor 702 may relay data and/or commands between interface unit 410 and cochlear implant 610.

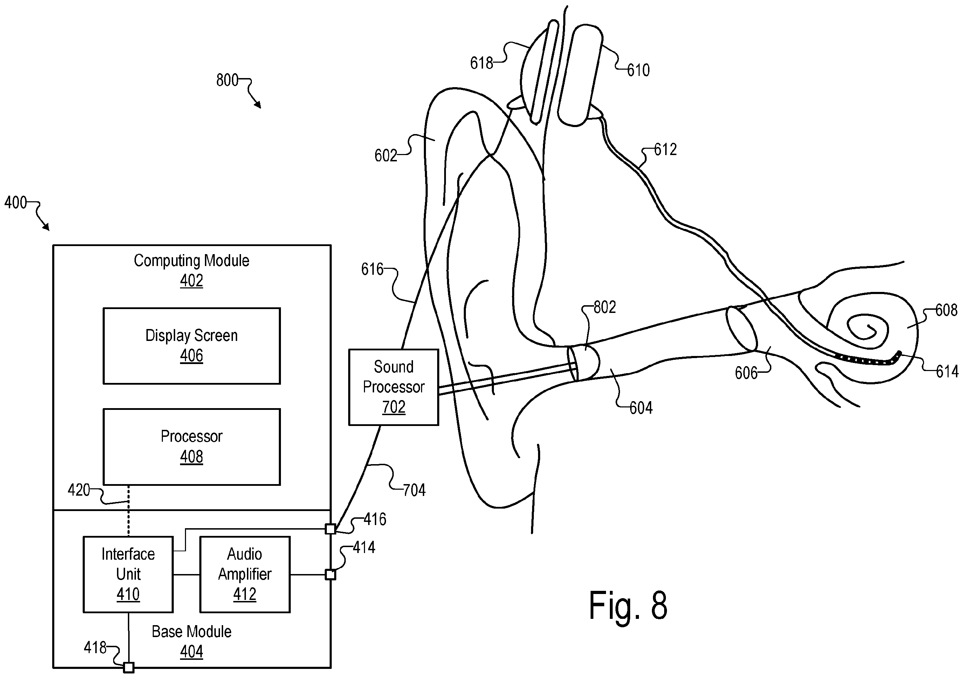

FIG. 8 illustrates an alternative configuration 800 in which sound processor 702 is configured to generate the acoustic stimulation that is applied to the recipient of cochlear implant 610. As shown, in this configuration, a sound delivery apparatus 802 is coupled directly to sound processor 702. For example, sound processor 702 may be implemented by a behind-the-ear bimodal sound processor and sound delivery apparatus 802 may be implemented by an audio ear hook that connects to sound processor 702.

It will be recognized that diagnostic system 400 may be additionally or alternatively implemented in any other suitable manner. For example, diagnostic system 400 may be implemented by a fitting system utilized in a clinician's office and/or by any other appropriately configured system or device.

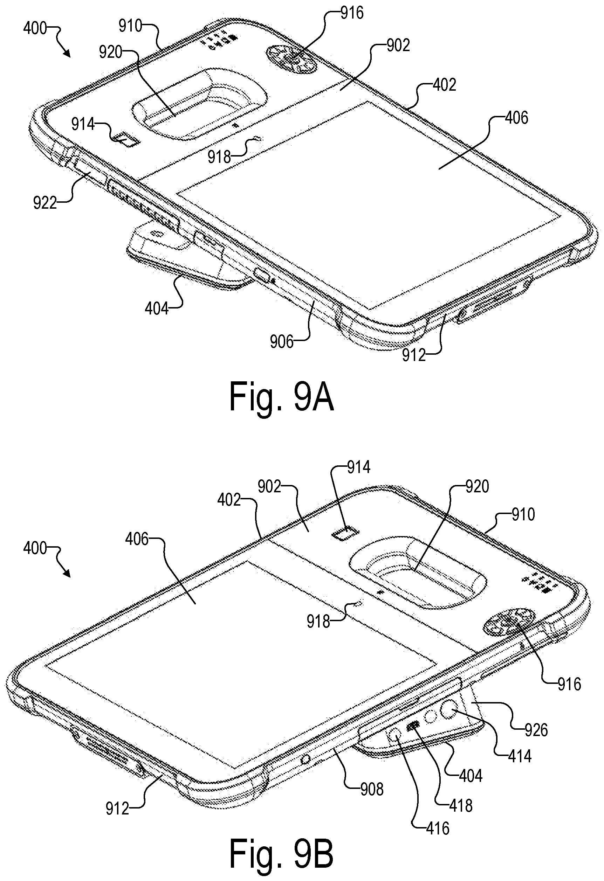

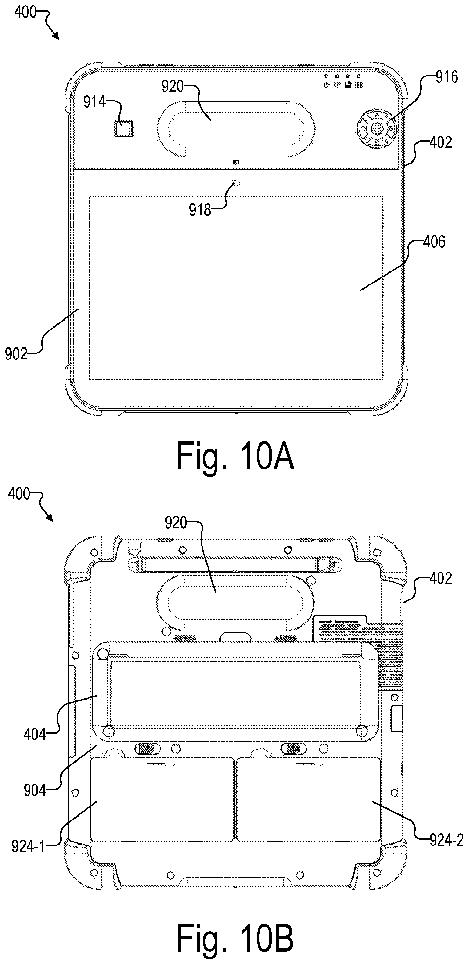

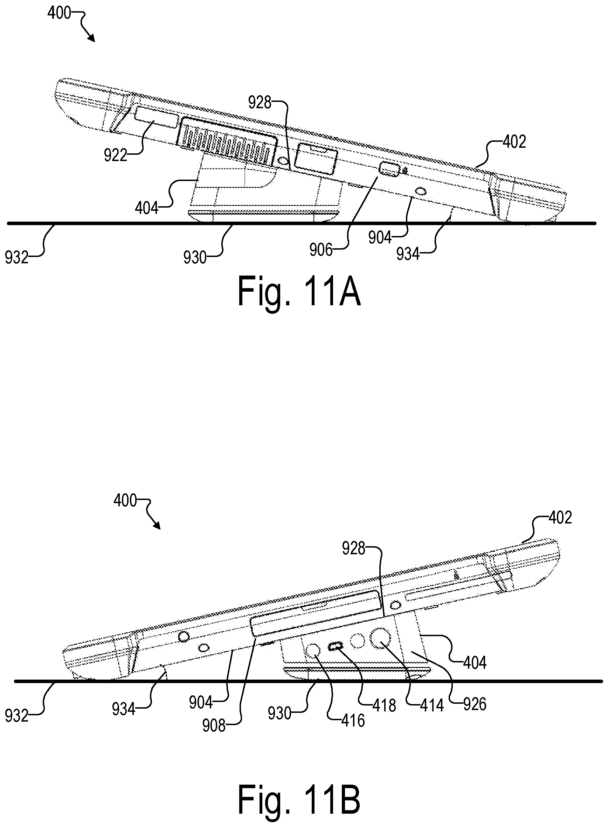

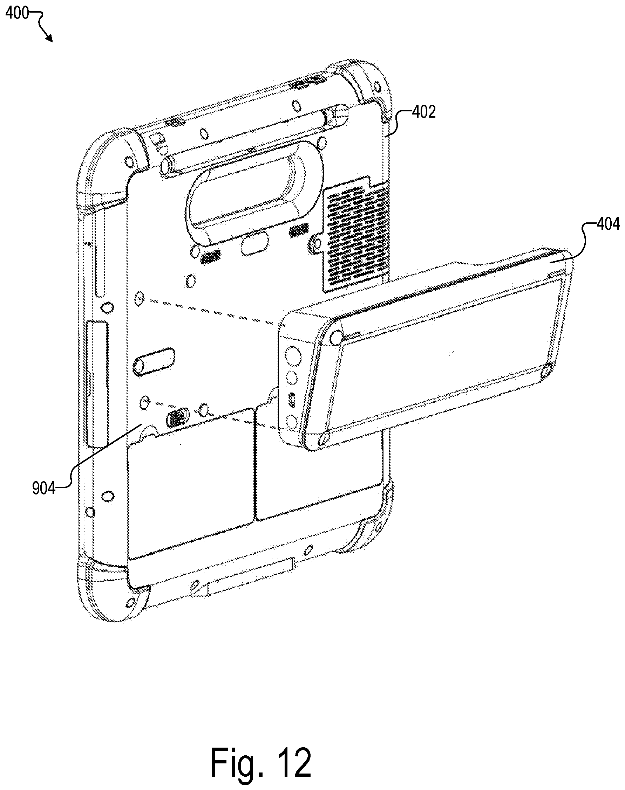

An exemplary hardware implementation of diagnostic system 400 will now be described in connection with FIGS. 9A-12. In particular, FIG. 9A shows a left perspective view of diagnostic system 400, FIG. 9B shows a right perspective view of diagnostic system 400, FIG. 10A shows a front view of diagnostic system 400, FIG. 10B shows a back view of diagnostic system 400, FIG. 11A shows a left side view of diagnostic system 400, FIG. 11B shows a right side view of diagnostic system 400, and FIG. 12 shows a rear perspective view of diagnostic system 400.

The hardware implementation of diagnostic system 400 illustrated in FIGS. 9A-12 includes computing module 402 and base module 404. As, illustrated computing module 402 includes a front side 902, a back side 904, a left side 906, a right side 908, a top side 910, and a bottom side 912.

Display screen 406 is located on front side 902 of computing module 402. Various other components are also located on the front side 902 of computing module 402. For example, a fingerprint scanner 914, physical input buttons 916, and a webcam 918 all shown to be included on the front side 902 of computing module 402. It will be recognized that any of these components may be located on any other side of computing module 402 as may serve a particular implementation.

Fingerprint scanner 914 is configured to facilitate authentication of a user of diagnostic system 400. For example, fingerprint scanner 914 may detect a fingerprint of the user and provide processor 408 with data representative of the fingerprint. Processor 408 may process the fingerprint data in any suitable manner (e.g., by comparing the fingerprint to known fingerprints included in a database) to authenticate the user.

Webcam 918 may be configured to facilitate video communication by a user of diagnostic system 400 with a remotely located user (e.g., during a surgical procedure). Such video communication may be performed in any suitable manner.

Physical input buttons 916 may be implemented, for example, by a directional pad and/or any other suitable type of physical input button. A user of diagnostic system 400 may interact with physical input buttons 916 to perform various operations with respect to a diagnostic application being executed by processor 408. For example, the user may use the physical input buttons 916 to interact with a graphical user interface displayed on display screen 406.

In some examples, physical input buttons 916 may be configured to be selectively programmed (e.g., as hotkeys) to perform one or more functions associated with the diagnostic application. For example, a particular physical input button 916 may be programmed by a user to start and/or stop acoustic stimulation being applied to a cochlear implant recipient by diagnostic system 400.

In some examples, processor 408 may be configured to wirelessly connect to an input device configured to be used by the user in connection with the diagnostic application. For example, processor 408 may be configured to wirelessly connect (e.g., via Bluetooth and/or any other suitable wireless communication protocol) to a keyboard, mouse, remote control, and/or any other wireless input device as may serve a particular implementation. In this manner, the user may selectively use physical input buttons 916, a touchscreen capability of display screen 406, and/or a wireless input device to interact with diagnostic system 400.

As shown, a hole 920 may be formed within computing module 402 and configured to serve as a handle for diagnostic system 400. A user may grip computing module 402 by placing his or her fingers within hole 920.

As shown, a barcode scanner 922 may be located on left side 906 of computing module 402. Barcode scanner 922 may alternatively be located on any other side of computing module 402. In some examples, barcode scanner 922 may be configured to scan for an activation code included on one or more components associated with a procedure being performed with respect to cochlear implant 510. The activation code may be used to associate (e.g., register) the components with cochlear implant 510.

As illustrated in FIG. 10B, computing module 402 may include batteries 924-1 and 924-2. Batteries 924 may be configured to provide operating power for various components included within computing module 402 and base module 404. In some examples, batteries 924 may be hot-swappable. In other words, one of batteries 924 (e.g., battery 924-1) may be removed and replaced while the other battery (e.g., battery 924-2) is used to provide power to computing module 402 and base module 404.

As illustrated in FIGS. 9B and 11B, ports 414, 416, and 418 are located on a side surface 926 of base module 404. Ports 414, 416, and 418 may alternatively be located on any other surface of base module 404.

As described above, base module 404 may be configured to serve as a stand for computing module 402 while base module 404 is attached to computing module 402. The stand functionality of base module 404 is illustrated in FIGS. 11A-11B.

As shown, base module 404 includes a top surface 928 configured to selectively attach to back side 904 of computing module 402. Base module 404 may alternatively attach to any other side of computing module 402. Base module 404 further includes a bottom surface 930 configured to be placed on a resting surface 932. Bottom surface 930 is angled with respect to back side 904 of computing module 402. This provides a viewing angle 934 for display screen 406 that is greater than zero degrees with respect to resting surface 932. In some examples, base module 404 may be adjustable to selectively provide different viewing angles for display screen 406 with respect to resting surface 932. This adjustability may be realized in any suitable manner. For example, a user may manually adjust bottom surface 930 to different angles with respect to back side 904 of computing module 402.

FIG. 12 illustrates an exemplary configuration in which base module 404 is detached from computing module 402. Base module 404 may be detached from computing module 402 in any suitable manner. For example, base module 404 may include one or more locking mechanisms that may be actuated by a user to detach base module 404 from computing module 402.

Various operations that may be performed by diagnostic system 300 will now be described. It will be recognized that diagnostic system 300 may perform additional or alternative operations to those described herein as may serve a particular implementation.

In some examples, diagnostic system 300 may determine a minimum evoked response amplitude and a maximum evoked response amplitude for a recipient of a cochlear implant. The minimum and maximum evoked response amplitudes define a range of evoked response amplitudes that are expected to occur within the recipient in response to acoustic stimulation applied to the recipient. Diagnostic system 300 may determine the minimum and maximum evoked response amplitudes in any suitable manner.

For example, as will be described in more detail below, diagnostic system 300 may present a graphical user interface by way of a display screen (e.g., display screen 406 or any other suitable display screen) and detect user input provided by way of the graphical user interface that is representative of the minimum and maximum evoked response amplitudes.

Additionally or alternatively, diagnostic system 300 may automatically determine the minimum and maximum evoked response amplitudes based on various characteristics of the acoustic stimulation that is to be applied to elicit the evoked responses, the recipient, and/or the electrode used to record the evoked responses. For example, the diagnostic system may automatically determine the minimum and maximum evoked response amplitudes based on a stimulation level (e.g., amplitude) of the acoustic stimulation that is to be applied to elicit the evoked responses, an age of the recipient, a pre-operative hearing assessment for the recipient, an impedance of the electrode, and/or any other suitable factor as may serve a particular implementation.

Once the minimum and maximum evoked response amplitudes have been determined, diagnostic system 300 may determine a mapping between a plurality of audible pitches and a plurality of evoked response amplitudes included in a range defined by the minimum and maximum evoked response amplitudes. For example, the plurality of audible pitches may include only a predetermined number of audible pitches (e.g., ten or fifteen different audible pitches). The lowest audible pitch within plurality of audible pitches may be mapped to the minimum evoked response amplitude. Likewise, the highest audible pitch within the plurality of audible pitches may be mapped to the maximum evoked response amplitude. A remaining number of available pitches within the plurality of audible pitches may be mapped to different evoked response amplitudes that are in between the minimum and maximum evoked response amplitudes.

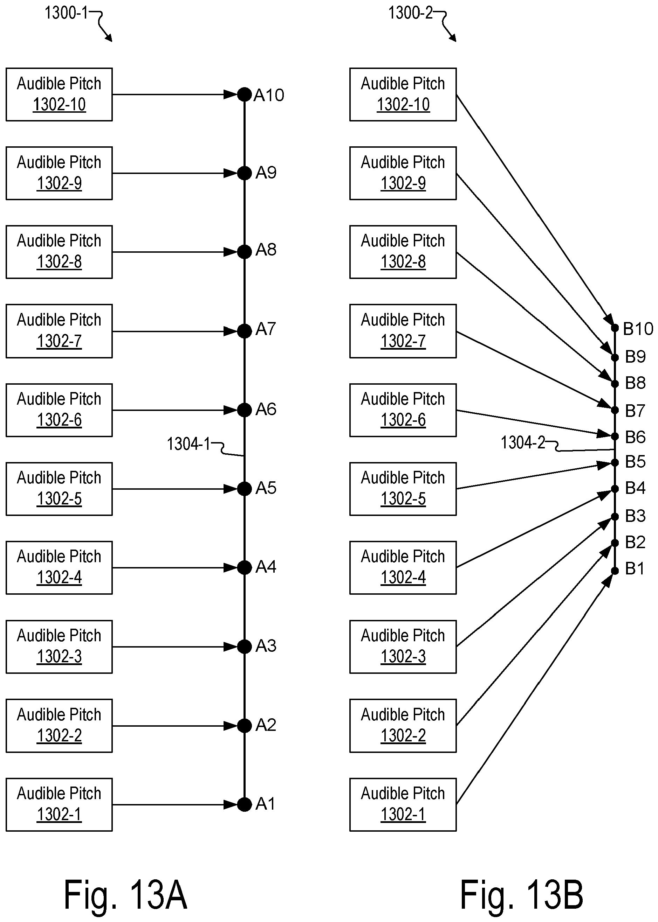

To illustrate, FIGS. 13A-13B illustrate two different mappings 1300-1 and 1300-2 that may be performed by diagnostic system 300. In mapping 1300-1, a total of ten audible pitches 1302-1 through 1302-10 (collectively "audible pitches 1302") are mapped to a first range of evoked response amplitudes represented by line 1304-1 and defined by a minimum evoked response amplitude A1 and a maximum evoked response amplitude A10. In mapping 1300-2, the same audible pitches 1302 are mapped to a second range of evoked response amplitudes represented by line 1304-2 and defined by a minimum evoked response amplitude B1 and a maximum evoked response amplitude B10. As illustrated by the relative lengths of lines 1304-1 and 1304-2, the first range of evoked response amplitudes is greater than the second range of evoked response amplitudes.

In FIG. 13A, the lowest audible pitch 1302-1 included in the plurality of audible pitches 1302 is mapped to the minimum evoked response amplitude A1 and the highest audible pitch 1302-10 included in the plurality of audible pitches 1302 is mapped to the maximum evoked response amplitude A10. The remaining audible pitches 1302-2 through 1302-9 are mapped to evoked response amplitudes A2 through A9, which are each included in the range defined by minimum evoked response amplitude A1 and maximum evoked response amplitude A10. In some examples, the mappings of audible pitches 1302-2 through 1302-9 are evenly distributed between the minimum and maximum evoked response amplitudes A1 and A10. The mapping of audible pitches 1302-2 through 1302-9 may alternatively be distributed in any suitable manner.

In FIG. 13B, the lowest audible pitch 1302-1 included in the plurality of audible pitches 1302 is mapped to the minimum evoked response amplitude B1 and the highest audible pitch 1302-10 included in the plurality of audible pitches 1302 is mapped to the maximum evoked response amplitude B10. The remaining audible pitches 1302-2 through 1302-9 are mapped to evoked response amplitudes B2 through B9, which are each included in the range defined by minimum evoked response amplitude B1 and maximum evoked response amplitude B10. In some examples, the mappings of audible pitches 1302-2 through 1302-9 are evenly distributed between the minimum and maximum evoked response amplitudes B1 and B10. The mapping of audible pitches 1302-2 through 1302-9 may alternatively be distributed in any suitable manner.

By using the same audible pitches 1302 in mappings 1300-1 and 1300-2, as well as all other mappings performed by diagnostic system 300, diagnostic system 300 may provide the same acoustic feedback experience to a user regardless of what the minimum and maximum evoked response amplitudes are set to be. This may allow a user to become accustomed to what the user should hear in terms of acoustic feedback as an electrode lead is inserted into the cochlea.

In some examples, audible pitches 1302 may be musically related one to another. For example, audible pitches 1302-1 through 1302-10 may each correspond to a musical note included in a musical scale. In this manner, the acoustic feedback may be pleasant to hear and allow the user to readily ascertain what audible pitch should be played at a particular point during an electrode lead insertion procedure.

During an insertion procedure in which an electrode lead communicatively coupled to a cochlear implant is inserted into a cochlea of a recipient, diagnostic system 300 may monitor an evoked response signal recorded during the insertion procedure by an electrode on the insertion lead. To this end, diagnostic system 300 may direct an acoustic stimulation generator to apply acoustic stimulation to the recipient during the insertion procedure. Diagnostic system 300 may also direct the cochlear implant to use an electrode disposed on the electrode lead to record the evoked response signal.

The acoustic stimulation generator may be implemented by interface unit 410, a behind-the-ear bimodal sound processor comprising an earhook (e.g., sound processor 702 shown in FIG. 8), and/or any other component configured to generate acoustic stimulation. For example, in configuration 600 shown in FIG. 6 and configuration 700 shown in FIG. 7, processor 408 implementing diagnostic system 300 may direct interface unit 410 to generate and apply acoustic stimulation to the recipient of cochlear implant 610 by way of sound delivery apparatus 620. As another example, in configuration 800 shown in FIG. 8, processor 408 implementing diagnostic system 300 may direct sound processor 702 to generate and apply acoustic stimulation to the recipient of cochlear implant 610 by way of sound delivery apparatus 802. In all of these configurations, processor 408 may direct cochlear implant 610 to use electrode 614 to record the evoked response signal. Use of electrode 614 to record the evoked response signal is beneficial in many configurations because electrode 614 is the first to enter the cochlea. However, it will be recognized that any other electrode disposed on electrode lead 612 may be used to record the evoked response signal.

Diagnostic system 300 may monitor the evoked response signal in any suitable manner. For example, diagnostic system 300 may monitor the evoked response signal by receiving data representative of the evoked response signal from the cochlear implant, analyzing the evoked response signal in real time during the insertion procedure, and performing various actions associated with the evoked response signal. For example, as will be described herein, diagnostic system 300 may plot the evoked response signal within a graphical user interface, provide acoustic feedback in real time as the evoked response signal is recorded, detect one or more events that occur within the evoked response signal, provide notifications of the one or more events, etc.

While diagnostic system 300 monitors the evoked response signal, diagnostic system 300 may detect an amplitude change in the evoked response signal and present acoustic feedback (e.g., by way of one or more speakers) that audibly indicates the amplitude change. To illustrate, the acoustic feedback may be based on mapping 1300-1. In this example, the evoked response amplitude may initially be A2. As such, diagnostic system 300 may initially present a tone that has audible pitch 1302-2. In response to a change in evoked response amplitude from A2 to A3, diagnostic system may present a tone that has audible pitch 1302-3. This change in audible pitch audibly indicates to a user that the evoked response amplitude has changed from A2 to A3.

In some examples, diagnostic system 300 may direct the display screen to display a graph of the evoked response signal as the evoked response signal is being recorded. In this manner, diagnostic system 300 may also graphically indicate amplitude changes that occur in the evoked response signal. Diagnostic system 300 may synchronize the presentation of the acoustic feedback and the display of the graph such that the acoustic feedback and the graph indicate the amplitude changes at substantially the same time. In this manner, a user may selectively rely on the acoustic feedback and/or the graph to monitor the evoked response signal.

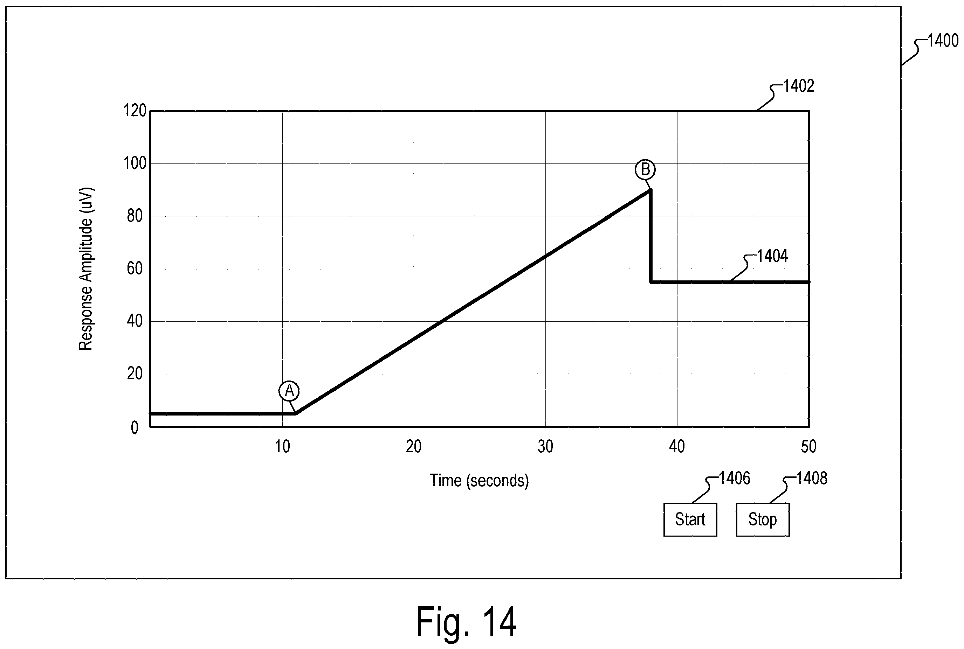

FIG. 14 illustrates an exemplary graphical user interface 1400 that may be presented by diagnostic system 300 by way of a display screen. As shown, graphical user interface 1400 includes a graph 1402 of an evoked response signal 1404 that may be recorded by an electrode during an electrode lead insertion procedure. As shown, graph 1402 plots the amplitude (y-axis) of the evoked response signal 1404 with respect to time (x-axis). While FIG. 14 shows evoked response signal 1404 plotted after 50 seconds, it will be recognized that the evoked response signal 1404 may be plotted in real time as the electrode insertion procedure occurs.

As shown, evoked response signal 1404 is initially flat prior to callout A (which is around 16 seconds in to the electrode lead insertion procedure). This may indicate that the electrode being used to record the evoked response signal 1404 has not yet reached the round window. However, between callout A and callout B (which is around 37 seconds in to the electrode lead insertion procedure), the evoked response signal 1404 increases in amplitude at a constant rate. This graphically indicates that the electrode lead is being properly inserted into the cochlea. As the evoked response signal 1404 increases in amplitude, diagnostic system 300 may present acoustic feedback that correspondingly increases in audible pitch, as described herein.

At callout B, there is a sudden drop in amplitude of evoked response signal 1404. In particular, the amplitude of evoked response signal 1404 drops from about 130 µV to about 57 µV. This sudden drop in evoked response amplitude may be indicative of an event that occurs during the electrode lead insertion procedure. For example, the sudden drop in evoked response amplitude may indicate that the electrode lead has penetrated or is otherwise damaging a wall of the cochlea. They event associated with the sudden drop may additionally or alternatively include any other type of event as may serve a particular implementation.

In response to the sudden drop in amplitude of evoked response signal 1404, diagnostic system 300 may correspondingly decrease the audible pitch of the acoustic feedback being presented to indicate the drop in amplitude. Additionally or alternatively, diagnostic system 300 may present a different type of acoustic feedback (referred to herein as event-based acoustic feedback) that specifically indicates the occurrence of an event.

For example, diagnostic system 300 may maintain data representative of an event threshold. The event threshold may be any suitable amount (e.g., in dB or µV) to which an evoked response amplitude change over a predetermined amount of time (e.g., a relatively short amount of time) may be compared in order to determine whether an event has occurred. For example, the event threshold may be 6 dB. In this example, if diagnostic system 300 determines that the amplitude of evoked response signal 1404 changes by at least 6 dB within a predetermined amount time, diagnostic system 300 may determine that an event has occurred and provide event-based acoustic feedback that is distinct from the acoustic feedback being used to generally indicate changes in the amplitude of evoked response signal 1404.

In some examples, the event-based acoustic feedback does not include any of the audible pitches included in the acoustic feedback used to generally indicate changes in the amplitude of evoked response signal 1404. For example, the event-based acoustic feedback may include one or more beeps over other alarm-like sounds. In some examples, the event-based acoustic feedback is presented concurrently with the acoustic feedback used to generally indicate changes in the amplitude of evoked response signal 1404.

While FIG. 14 illustrates a sudden drop in amplitude of evoked response signal 1404, it will be recognized that other types of events may be associated with sudden increases in amplitude of evoked response signal 1404. Acoustic feedback representative of the sudden increases in amplitude may be presented to the user in a similar manner.

In some examples, a sudden change in phase of an evoked response signal recorded by the electrode may additionally or alternatively be used to determine that an event, such as damage to the cochlea, has occurred. This is described in more detail in WO2017/131675, which application is incorporated herein by reference in its entirety. Hence, in some examples, diagnostic system 300 may track the phase of the evoked response signal and provide acoustic feedback if the phase changes more than a threshold amount (e.g., more than 3 radians) over a predetermined time period. In some alternative examples, the acoustic feedback may be provided if the amplitude changes more than a threshold amount without the phase changing.

As shown in FIG. 14, graphical user interface 1400 may include a start option 1406 and a stop option 1408 displayed therein. A user may interact with these options to direct diagnostic system 300 to begin and stop monitoring evoked response signal 1404. For example, graphical user interface 1400 may detect a selection of start option 1406. In response, diagnostic system 300 may begin monitoring evoked response signal 1404. While monitoring evoked response signal 1404, diagnostic system 300 may detect a user selection of stop option 1408. In response, diagnostic system 300 may stop monitoring evoked response signal 1404.

In some examples, in response to detecting a user selection of start option 1406 (or any other command that directs diagnostic system 300 to begin monitoring evoked response signal 1404), diagnostic system 300 may measure an impedance of the electrode being used to record evoked response signal 1404 and abstain from beginning to monitor evoked response signal 1404 until the impedance of the electrode is below a predetermined threshold. This below threshold impedance of the electrode may indicate that the electrode is touching the round window within the ear of the recipient. At this point, diagnostic system 300 may begin applying acoustic stimulation to the recipient and monitoring evoked response signal 1404 that occurs in response to the acoustic stimulation.

To illustrate, in the example of FIG. 14, start option 1406 was selected by the user at time equals zero seconds. In response, diagnostic system 300 began measuring the impedance of the electrode used to record evoked response signal 1404. At the time associated with callout A, diagnostic system 300 determined that the impedance of the electrode went below the predetermined threshold. Between time zero and the time associated with callout A, diagnostic system 300 abstained from monitoring evoked response signal 1404 (e.g., by abstaining from presenting acoustic stimulation to the recipient during this time period). At the time associated with callout A, diagnostic system 300 began applying the acoustic stimulation to the recipient and monitoring the resultant evoked response signal 1404.

In some examples, diagnostic system 300 may be configured to use multi-rate analysis to detect amplitude changes in evoked response signal 1404 and present acoustic feedback indicating the amplitude changes. For example, diagnostic system 300 may employ a plurality of averagers in parallel that each average a different number of samples (e.g., one, two, four, eight, sixteen, and thirty-two samples) of evoked response signal 1404. If any averager detects a change in evoked response signal 1404, diagnostic system 300 may plot the change within graph 1402. The averagers are then all reset to be maximally sensitive to the next change. Diagnostic system 300 may be configured to ignore data where there is motion or other artifact from the change analysis. By using multi-rate analysis, diagnostic system 300 may detect changes in amplitude of evoked response signal 1404 at an optimal rate.

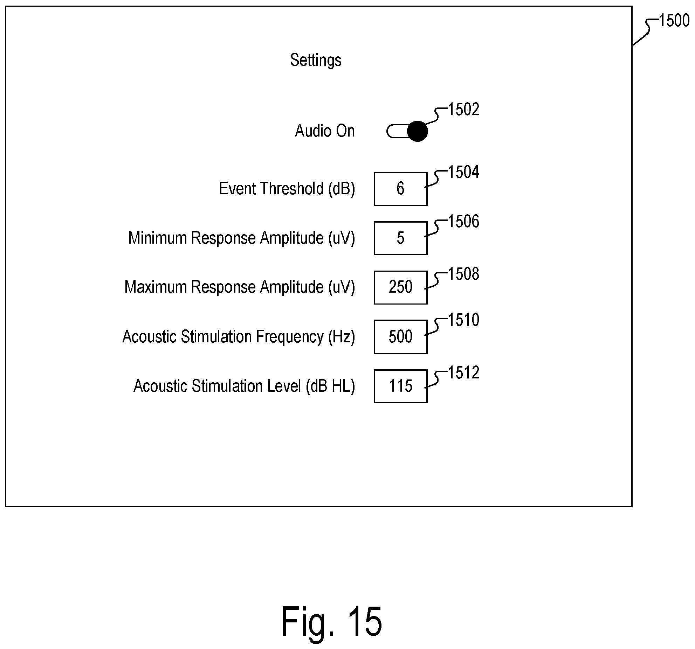

FIG. 15 illustrates an exemplary graphical user interface 1500 that may be presented by diagnostic system 300 and that may facilitate user control of various settings associated with monitoring evoked responses that occur during an electrode lead insertion procedure.

For example, a user may interact with a slider 1502 to selectively enable or disable acoustic feedback during the electrode lead insertion procedure. In the example of FIG. 15, the position of slider 1502 indicates that acoustic feedback is to be provided during the electrode lead insertion procedure.

The user may additionally or alternatively interact with field 1504 to provide user input representative of an event threshold. In the example of FIG. 15, the event threshold is set to 6 dB, which, as described above, means that if a change in evoked response amplitude is greater than 6 dB (e.g., between subsequent evoked response amplitude samplings or during a particular time period), diagnostic system 300 may determine that an event has occurred and provide acoustic feedback representative of the event.

The user may additionally or alternatively interact with fields 1506 and 1508 to provide user input that sets the minimum evoked response amplitude and the maximum evoked response amplitude. As shown, the minimum evoked response amplitude is set to 5 microvolts (µV) and the maximum evoked response amplitude is set to 250 µV. As described above, the minimum and maximum evoked response amplitudes define a range of evoked response amplitudes that are expected to occur within the recipient.

The user may additionally or alternatively interact with fields 1510 and 1512 to provide user input that specifies a frequency and stimulation level, respectively, of the acoustic stimulation that is applied to the recipient to elicit evoked responses. In the example of FIG. 15, the acoustic stimulation frequency is set to 500 Hz and the acoustic stimulation level is set to 115 dB HL.

In some examples, diagnostic system 300 may be configured to perform any of the operations described herein while operating in a "demo" mode. In the demo mode, instead of performing operations with respect to an actual recipient of a cochlear implant, diagnostic system 300 may perform the operations with respect to one or more recipient models. In this manner, diagnostic system 300 may readily provide opportunities for user training.

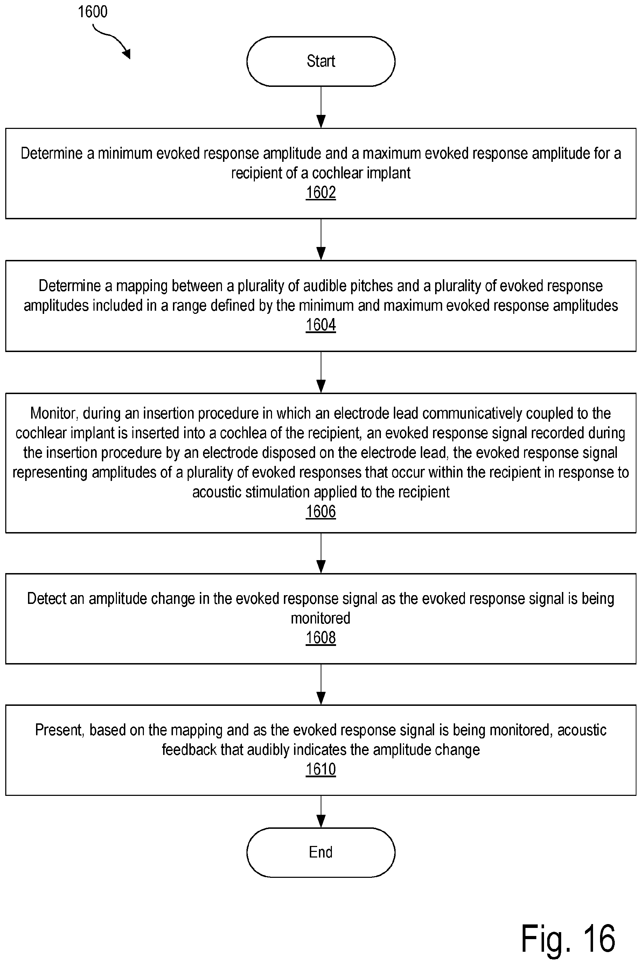

FIG. 16 illustrates an exemplary method 1600. The operations shown in FIG. 16 may be performed by diagnostic system 300 and/or any implementation thereof. While FIG. 16 illustrates exemplary operations according to one embodiment, other embodiments may omit, add to, reorder, and/or modify any of the operations shown in FIG. 16.

In operation 1602, a diagnostic system determines a minimum evoked response amplitude and a maximum evoked response amplitude for a recipient of a cochlear implant. Operation 1602 may be performed in any of the ways described herein.

In operation 1604, the diagnostic system determines a mapping between a plurality of audible pitches and a plurality of evoked response amplitudes included in a range defined by the minimum and maximum evoked response amplitudes. Operation 1604 may be performed in any of the ways described herein.

In operation 1606, the diagnostic system monitors, during an insertion procedure in which an electrode lead communicatively coupled to the cochlear implant is inserted into a cochlea of the recipient, an evoked response signal recorded during the insertion procedure by an electrode disposed on the electrode lead, the evoked response signal representing amplitudes of a plurality of evoked responses that occur within the recipient in response to acoustic stimulation applied to the recipient. Operation 1606 may be performed in any of the ways described herein.

In operation 1608, the diagnostic system detects an amplitude change in the evoked response signal as the evoked response signal is being monitored. Operation 1608 may be performed in any of the ways described herein.

In operation 1610, the diagnostic system presents, based on the mapping and as the evoked response signal is being monitored, acoustic feedback that audibly indicates the amplitude change. Operation 1610 may be performed in any of the ways described herein.

In some examples, a non-transitory computer-readable medium storing computer-readable instructions may be provided in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Exemplary non-volatile storage media include, but are not limited to, read-only memory, flash memory, a solid-state drive, a magnetic storage device (e.g. a hard disk, a floppy disk, magnetic tape, etc.), ferroelectric random-access memory ("RAM"), and an optical disc (e.g., a compact disc, a digital video disc, a Blu-ray disc, etc.). Exemplary volatile storage media include, but are not limited to, RAM (e.g., dynamic RAM).

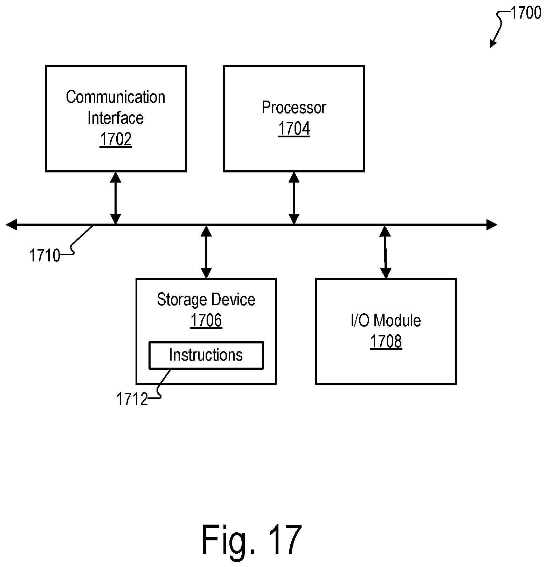

FIG. 17 illustrates an exemplary computing device 1700 that may be specifically configured to perform one or more of the processes described herein. As shown in FIG. 17, computing device 1700 may include a communication interface 1702, a processor 1704, a storage device 1706, and an input/output ("I/O") module 1708 communicatively connected one to another via a communication infrastructure 1710. While an exemplary computing device 1700 is shown in FIG. 17, the components illustrated in FIG. 17 are not intended to be limiting. Additional or alternative components may be used in other embodiments. Components of computing device 1700 shown in FIG. 17 will now be described in additional detail.

Communication interface 1702 may be configured to communicate with one or more computing devices. Examples of communication interface 1702 include, without limitation, a wired network interface (such as a network interface card), a wireless network interface (such as a wireless network interface card), a modem, an audio/video connection, and any other suitable interface.

Processor 1704 generally represents any type or form of processing unit capable of processing data and/or interpreting, executing, and/or directing execution of one or more of the instructions, processes, and/or operations described herein. Processor 1704 may perform operations by executing computer-executable instructions 1712 (e.g., an application, software, code, and/or other executable data instance) stored in storage device 1706.

Storage device 1706 may include one or more data storage media, devices, or configurations and may employ any type, form, and combination of data storage media and/or device. For example, storage device 1706 may include, but is not limited to, any combination of the non-volatile media and/or volatile media described herein. Electronic data, including data described herein, may be temporarily and/or permanently stored in storage device 1706. For example, data representative of computer-executable instructions 1712 configured to direct processor 1704 to perform any of the operations described herein may be stored within storage device 1706. In some examples, data may be arranged in one or more databases residing within storage device 1706.

I/O module 1708 may include one or more I/O modules configured to receive user input and provide user output. I/O module 1708 may include any hardware, firmware, software, or combination thereof supportive of input and output capabilities. For example, I/O module 1708 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1708 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1708 is configured to provide graphical data to a display for presentation to a user. The graphical data may be representative of one or more graphical user interfaces and/or any other graphical content as may serve a particular implementation.

In some examples, any of the systems, computing devices, and/or other components described herein may be implemented by computing device 1700. For example, storage facility 302 may be implemented by storage device 1706, and processing facility 304 may be implemented by processor 1704.

The present invention is set out in the appended claims that follow.

## Claims

1. A system comprising:
a memory (302, 1706) storing instructions (306, 1712);
a processor (304, 408, 1704) communicatively coupled to the memory and configured to execute the instructions to:
determine a minimum evoked response amplitude and a maximum evoked response amplitude for a recipient of a cochlear implant (108, 610);
determine a mapping (1300) between a plurality of audible pitches (1302) and a plurality of evoked response amplitudes (1304) included in a range defined by the minimum and maximum evoked response amplitudes;
monitor, during an insertion procedure in which an electrode lead (110, 612) communicatively coupled to the cochlear implant is inserted into a cochlea (200) of the recipient, an evoked response signal (1404) recorded during the insertion procedure by an electrode (112, 614) disposed on the electrode lead, the evoked response signal representing amplitudes of a plurality of evoked responses that occur within the recipient in response to acoustic stimulation applied to the recipient;
detect an amplitude change in the evoked response signal as the evoked response signal is being monitored; and
present, based on the mapping and as the evoked response signal is being monitored, acoustic feedback that audibly indicates the amplitude change,
wherein the determining of the mapping comprises:
mapping a lowest audible pitch within the plurality of audible pitches to the minimum evoked response amplitude;
mapping a highest audible pitch within the plurality of audible pitches to the maximum evoked response amplitude; and
mapping a remaining number of available pitches within the plurality of audible pitches to different evoked response amplitudes that are in between the minimum and maximum evoked response amplitudes.

2. The system of claim 1, wherein the processor (304, 408, 1704) is further configured to execute the instructions (306, 1712) to:
direct a display screen (406, 1400) to display a graph (1402) of the evoked response signal (1404) that graphically indicates the amplitude change; and
synchronize the presentation of the acoustic feedback and the display of the graph such that the acoustic feedback and the graph indicate the amplitude change at substantially the same time.

3. The system of claim 1, wherein the processor (304, 408, 1704) is further configured to execute the instructions (306, 1712) to:
determine that the amplitude change is greater than an event threshold associated with an event; and
present, in response to the amplitude change being greater than the event threshold, additional acoustic feedback that is distinct from the acoustic feedback and that does not include any of the plurality of audible pitches (1302), the additional acoustic feedback audibly indicating an occurrence of the event.

4. The system of claim 3, wherein the additional acoustic feedback is presented concurrently with the acoustic feedback.

5. The system of claim 3, wherein the processor (304, 408, 1704) is further configured to execute the instructions (306, 1712) to:
present a graphical user interface (1400) by way of a display screen (406); and
detect user input provided by way of the graphical user interface and that specifies the event threshold.

6. The system of claim 1, wherein:
the processor (304, 408, 1704) is further configured to execute the instructions (306, 1712) to present a graphical user interface (1400) by way of a display screen (406); and
the determining of the minimum and maximum evoked response amplitudes (1304) comprises detecting user input provided by way of the graphical user interface and representative of the minimum and maximum evoked response amplitudes.

7. The system of claim 1, wherein the audible pitches (1302) are musically related to each other, with the audible pitches each corresponding to a musical note included in a musical scale.

8. The system of claim 1, wherein the processor (304, 408, 1704) is further configured to execute the instructions (306, 1712) to:
direct an acoustic stimulation generator (408, 620, 702, 802) to apply the acoustic stimulation; and
direct the cochlear implant (108, 610) to use the electrode (112, 614) to record the evoked response signal (1404).

9. The system of claim 8, wherein:
the processor (304, 408, 1704) is included in a computing module (402) of a stand-alone diagnostic system (300, 400); and
the acoustic stimulation generator comprises an interface unit (410) included in a base module (404) configured to be attached to the computing module.

10. The system of claim 8, wherein the acoustic stimulation generator is implemented by a behind-the-ear bimodal sound processor (702) comprising an audio earhook (802).

11. The system of claim 1, wherein the evoked responses are ECochG potentials.

12. The system of claim 1, wherein the processor (304, 408, 1704) is further configured to execute the instructions (306, 1712) to:
present a graphical user interface (1400) by way of a display screen (406);
detect a selection of a start option displayed within the graphical user interface; and
begin the monitoring of the evoked response signal (1404) in response to the selection of the start option.

13. The system of claim 12, wherein the processor (304, 408, 1704) is further configured to execute the instructions (306, 1712) to:
measure an impedance of the electrode (in response to the selection of the start option; and
abstain from beginning to monitor the evoked response signal until the impedance of the electrode (112, 614) is below a predetermined threshold.

14. The system of claim 1, wherein the processor (304, 408, 1704) is further configured to execute the instructions (306, 1712) to use multi-rate analysis to detect the amplitude change and present the acoustic feedback.

## Patentansprüche

1. Ein System mit:
einem Speicher (302, 1706) zum Speichern von Befehlen (306, 1712);
einem Prozessor (304, 408, 1704), der kommunikativ mit dem Speicher gekoppelt ist und ausgebildet ist, um die Befehle auszuführen, um:
eine minimale evozierte Antwortamplitude und eine maximale evozierte Antwortamplitude für einen Empfänger eines Cochlea-Implantats (108, 610) zu bestimmen;
eine Zuordnung (1300) zwischen einer Mehrzahl von hörbaren Tonhöhen (1302) und einer Mehrzahl von evozierten Antwortamplituden (1304) zu bestimmen, die in einem von der minimalen evozierten Antwortamplitude und der maximalen evozierten Antwortamplitude definierten Bereich liegen;
während einer Einführprozedur, in welcher eine Elektrodenzuleitung (110, 612), die kommunikativ mit dem Cochlea-Implantat gekoppelt ist, in eine Cochlea (200) des Empfängers eingeführt wird, ein evoziertes Antwortsignal zu überwachen, welches während der Einführprozedur von einer auf der Elektrodenzuleitung angeordneten Elektrode (112, 614) aufgezeichnet wird und welches Amplituden einer Mehrzahl von evozierten Antworten repräsentiert, welche innerhalb des Empfängers als Antwort auf auf den Empfänger angewandte akustische Stimulation auftreten;
eine Amplitudenveränderung in dem evozierten Antwortsignal während der Überwachung des evozierten Antwortsignals zu erfassen; und
basierend auf der Zuordnung und während dem Überwachen des evozierten Antwortsignals akustische Rückmeldung, welche hörbar die Amplitudenveränderung anzeigt, darzubieten,
wobei das Bestimmen der Zuordnung Folgendes umfasst:
Zuordnung einer tiefsten hörbaren Tonhöhe innerhalb der Mehrzahl von hörbaren Tonhöhen zu der minimalen evozierten Antwortamplitude;
Zuordnen einer höchsten hörbaren Tonhöhe innerhalb der Mehrzahl von hörbaren Tonhöhen zu der maximalen evozierten Antwortamplitude; und
Zuordnen einer verbleibenden Anzahl von verfügbaren Tonhöhen innerhalb der Mehrzahl von hörbaren Tonhöhen zu unterschiedlichen evozierten Antwortamplituden, welche zwischen der minimalen und der maximalen evozierten Antwortamplitude liegen.

2. System gemäß Anspruch 1, wobei der Prozessor (304, 408, 1704) ferner ausgebildet ist, um die Befehle (306, 1712) auszuführen, um:
einen Anzeigeschirm (406, 1400) zu veranlassen, einen Graphen (1402) des evozierten Antwortsignals (1404) anzuzeigen, welcher graphisch die Amplitudenveränderung anzeigt; und
die Darbietung der akustischen Rückmeldung und die Anzeige des Graphen so zu synchronisieren, dass die akustische Rückmeldung und der Graph die Amplitudenveränderung im Wesentlichen zum selben Zeitpunkt angeben.

3. System gemäß Anspruch 1, wobei der Prozessor (304, 408, 1704) ferner ausgebildet ist, um die Befehle (306, 1712) auszuführen, um:
festzustellen, dass die Amplitudenveränderung größer als eine Ereignisschwelle ist, welche mit einem Ereignis assoziiert ist; und
als Reaktion darauf, dass die Amplitudenveränderung größer als die Ereignisschwelle ist, zusätzliche akustische Rückmeldung darzubieten, welche sich von der akustischen Rückmeldung unterscheidet und keine der Mehrzahl von hörbaren Tonhöhen (1302) beinhaltet, wobei die zusätzliche akustische Rückmeldung hörbar ein Auftreten des Ereignisses angibt.

4. System gemäß Anspruch 3, wobei die zusätzliche akustische Rückmeldung gleichzeitig mit der akustischen Rückmeldung dargeboten wird.

5. System gemäß Anspruch 3, wobei der Prozessor (304, 408, 1704) ferner ausgebildet ist, um die Befehle (306, 1712) auszuführen, um:
eine graphische Benutzeroberfläche (1400) mittels eines Anzeigeschirms (406) darzubieten; und
eine Benutzereingabe zu erfassen, die mittels der graphischen Benutzeroberfläche bereitgestellt wird und die Ereignisschwelle spezifiziert.

6. System gemäß Anspruch 1, wobei:
der Prozessor (304, 408, 1704) ferner ausgebildet ist, um die Befehle (306, 1712) auszuführen, um eine graphische Benutzeroberfläche (1400) mittels eines Anzeigeschirms (406) darzubieten; und
das Bestimmen der minimalen und maximalen evozierten Antwortamplitude (1304) das Erfassen einer Nutzereingabe umfasst, die mittels der graphischen Benutzeroberfläche bereitgestellt wird und repräsentativ für die minimale und maximale evozierte Antwortamplitude ist.

7. System gemäß Anspruch 1, wobei die hörbaren Tonhöhen (1302) musikalisch in Beziehung miteinander stehen und jeweils einer musikalischen Note, die in einer musikalischen Skala beinhaltet ist, entsprechen.

8. System gemäß Anspruch 1, wobei der Prozessor (304, 408, 1704) ferner ausgebildet ist, um die Befehle (306, 1712) auszuführen, um:
einen akustischen Stimulationsgenerator (408, 620, 702, 802) zu veranlassen, die akustische Stimulation anzuwenden; und
das Cochlea-Implantat (108, 610) zu veranlassen, die Elektrode (112, 614) zu verwenden, um das evozierte Antwortsignal (1404) aufzuzeichnen.

9. System gemäß Anspruch 8, wobei:
der Prozessor (304, 408, 1704) in einem Berechnungsmodul (402) eines autarken Diagnosesystems (300, 400) beinhaltet ist; und
der akustische Stimulationsgenerator eine Schnittstelleneinheit (410) aufweist, die in einem Basismodul (404) beinhaltet ist, das ausgebildet ist, um an dem Berechnungsmodul befestigt zu werden.

10. System gemäß Anspruch 8, wobei der akustische Stimulationsgenerator mittels eines hinter-dem-Ohr Bimodal-Schallprozessor (702) mit einem Audioohrhaken (802) implementiert ist.

11. System gemäß Anspruch 1, wobei es sich bei den evozierten Antworten um ECochG-Potentiale handelt.

12. System gemäß Anspruch 1, wobei der Prozessor (304, 408, 1704) ferner ausgebildet ist, um die Befehle (306, 1712) auszuführen, um:
eine graphische Benutzeroberfläche (1400) mittels eines Anzeigeschirms (406) darzubieten;
eine Auswahl einer innerhalb der graphischen Benutzeroberfläche angezeigten Startoption zu erfassen; und
das Überwachen des evozierten Antwortsignals (1404) als Reaktion auf die Auswahl der Startoption zu beginnen.

13. System gemäß Anspruch 12, wobei der Prozessor (304, 408, 1704) ferner ausgebildet ist, um die Befehle (306, 1712) auszuführen, um:
eine Impedanz der Elektrode als Reaktion auf die Auswahl der Startoption zu messen; und
das Beginnen des Überwachens des evozierten Antwortsignals zu vermeiden, bis die Impedanz der Elektrode (112, 614) unterhalb eines vorbestimmten Schwellwerts liegt.

14. System gemäß Anspruch 1, wobei der Prozessor (304, 408, 1704) ferner ausgebildet ist, um die Befehle (306, 1712) auszuführen, um eine Multi-Ratenanalyse zu verwenden, um die Amplitudenveränderung zu erfassen und die akustische Rückmeldung darzubieten.

## Revendications

1. Système comprenant :
une mémoire (302, 1706) stockant des instructions (306, 1712) ;
un processeur (304, 408, 1704) couplé de façon communicante à la mémoire et configuré pour exécuter les instructions pour :
déterminer une amplitude minimale de potentiel évoqué et une amplitude maximale de potentiel évoqué pour un receveur d'un implant cochléaire (108, 610) ;
déterminer une mise en correspondance (1300) entre une pluralité de hauteurs audibles (1302) et une pluralité d'amplitudes de potentiel évoqué (1304) comprises dans une plage définie par les amplitudes minimale et maximale de potentiel évoqué ;
surveiller, pendant une procédure d'insertion dans laquelle un fil d'électrode (110, 612) couplé de façon communicante à l'implant cochléaire est inséré dans une cochlée (200) du receveur, un signal de potentiel évoqué (1404) enregistré pendant la procédure d'insertion par une électrode (112, 614) disposée sur le fil d'électrode, le signal de potentiel évoqué représentant les amplitudes d'une pluralité de potentiels évoqués qui apparaissent dans le corps du receveur en réponse à une stimulation acoustique appliquée au receveur ;
détecter un changement d'amplitude dans le signal de potentiel évoqué pendant que le signal de potentiel évoqué est surveillé ; et
présenter, sur la base de la mise en correspondance et pendant que le signal de potentiel évoqué est surveillé, un retour acoustique qui indique de façon audible le changement d'amplitude,
dans lequel la détermination de la mise en correspondance comprend :
la mise en correspondance d'une hauteur audible la plus basse au sein de la pluralité de hauteurs audibles avec l'amplitude minimale de potentiel évoqué ;
la mise en correspondance d'une hauteur audible la plus haute au sein de la pluralité de hauteurs audibles avec l'amplitude maximale de potentiel évoqué ; et
la mise en correspondance d'un nombre restant de hauteurs disponibles au sein de la pluralité de hauteurs audibles avec différentes amplitudes de potentiel évoqué qui se situent entre les amplitudes minimale et maximale de potentiel évoqué.

2. Système selon la revendication 1, dans lequel le processeur (304, 408, 1704) est également configuré pour exécuter les instructions (306, 1712) pour :
commander un écran d'affichage (406, 1400) pour afficher un graphe (1402) du signal de potentiel évoqué (1404) qui indique graphiquement le changement d'amplitude ; et
synchroniser la présentation du retour acoustique et l'affichage du graphe de telle sorte que le retour acoustique et le graphe indiquent le changement d'amplitude sensiblement en même temps.

3. Système selon la revendication 1, dans lequel le processeur (304, 408, 1704) est également configuré pour exécuter les instructions (306, 1712) pour :
déterminer que le changement d'amplitude est supérieur à un seuil d'événement associé à un événement ; et
présenter, en réponse au fait que le changement d'amplitude est supérieur au seuil d'événement, un retour acoustique supplémentaire qui est distinct du retour acoustique et qui ne comporte aucune de la pluralité de hauteurs audibles (1302), le retour acoustique supplémentaire indiquant de façon audible une occurrence de l'événement.

4. Système selon la revendication 3, dans lequel le retour acoustique supplémentaire est présenté simultanément au retour acoustique.

5. Système selon la revendication 3, dans lequel le processeur (304, 408, 1704) est également configuré pour exécuter les instructions (306, 1712) pour :
présenter une interface utilisateur graphique (1400) à l'aide d'un écran d'affichage (406) ; et
détecter une entrée utilisateur fournie à l'aide de l'interface utilisateur graphique et qui spécifie le seuil d'événement.

6. Système selon la revendication 1, dans lequel :
le processeur (304, 408, 1704) est également configuré pour exécuter les instructions (306, 1712) pour présenter une interface utilisateur graphique (1400) à l'aide d'un écran d'affichage (406) ; et
la détermination des amplitudes minimale et maximale de potentiel évoqué (1304) comprend la détection d'une entrée utilisateur fournie à l'aide de l'interface utilisateur graphique et représentative des amplitudes minimale et maximale de potentiel évoqué.

7. Système selon la revendication 1, dans lequel les hauteurs audibles (1302) sont associées musicalement les unes aux autres, les hauteurs audibles correspondant chacune à une note de musique comprise dans une échelle musicale.

8. Système selon la revendication 1, dans lequel le processeur (304, 408, 1704) est également configuré pour exécuter les instructions (306, 1712) pour :
commander un générateur de stimulations acoustiques (408, 620, 702, 802) pour appliquer la stimulation acoustique ; et
commander l'implant cochléaire (108, 610) pour utiliser l'électrode (112, 614) pour enregistrer le signal de potentiel évoqué (1404).

9. Système selon la revendication 8, dans lequel :
le processeur (304, 408, 1704) est contenu dans un module informatique (402) d'un système de diagnostic autonome (300, 400) ; et
le générateur de stimulations acoustiques comprend une unité d'interface (410) contenue dans un module de base (404) configuré pour être attaché au module informatique.

10. Système selon la revendication 8, dans lequel le générateur de stimulations acoustiques est mis en oeuvre par un processeur sonore bimodal en contour d'oreille (702) comprenant un coude audio (802).

11. Système selon la revendication 1, dans lequel les potentiels évoqués sont des potentiels ECochG.

12. Système selon la revendication 1, dans lequel le processeur (304, 408, 1704) est également configuré pour exécuter les instructions (306, 1712) pour :
présenter une interface utilisateur graphique (1400) à l'aide d'un écran d'affichage (406) ;
détecter une sélection d'une option de démarrage affichée au sein de l'interface utilisateur graphique ; et
lancer la surveillance du signal de potentiel évoqué (1404) en réponse à la sélection de l'option de démarrage.

13. Système selon la revendication 12, dans lequel le processeur (304, 408, 1704) est également configuré pour exécuter les instructions (306, 1712) pour :
mesurer une impédance de l'électrode en réponse à la sélection de l'option de démarrage ; et
s'abstenir de lancer la surveillance du signal de potentiel évoqué tant que l'impédance de l'électrode (112, 614) n'est pas inférieure à un seuil prédéterminé.

14. Système selon la revendication 1, dans lequel le processeur (304, 408, 1704) est également configuré pour exécuter les instructions (306, 1712) pour utiliser une analyse multicadence pour détecter le changement d'amplitude et présenter le retour acoustique.
